# EUROPEAN PATENT APPLICATION

(11) **EP 1 918 715 A1**
(43) Date of publication of application: **07.05.2008**
(21) Application number: 06794042.9
(22) Date of filing: 22.06.2006
(51) Int. Cl.: G01N 33/68

(54) **FIBROSIS MARKERS**

(30) Priority: 01.07.2005 ES 200501611
(71) Applicant: Proyecto de Biomedicina Cima, S.L., 31180 Cizur Mayor (Navarra) (ES)
(72) Inventor: SESMA AGUIRRE, Laura, Avda Pío XII, 55, 31008 Pamplona (ES); FERNANDEZ IRIGOYEN, Joaquín, Avda Pío XII, 55, 31008 Pamplona (ES); PRIETO VALTUEÑA, Jesús, Avda Pío XII, 55, 31008 Pamplona (ES); AVILA ZARAGOZA, Matías, Avda Pío XII, 55, 31008 Pamplona (ES)
(74) Representative: Ungria Lopez, Javier
(86) International application number: PCT/ES2006/000367
(87) International publication number: WO 2007/003670

(57) **Abstract**

The present invention the present invention relates to the use of a combination of at least two markers selected from uromodulin, MAC2BP, AGP1 and cathepsin A, for the in vitro detection of fibrotic alterations. Further, the present invention relates also to a kit for the determination of the levels of said markers in a biological sample.

## Description

### DESCRIPTION

The present invention relates to the use of biological markers for fibrosis identification.

### PRIOR ART

Fibrotic alterations or diseases constitute one of the main causes of morbidity/mortality and their chronic nature has an effect on the patients and society with considerable financial burdens. The progressive fibrosis of organs and tissues, including the liver, lungs, kidneys, heart, blood vessels and the skin, comprise a constellation of mechanistically related alterations. Each one of these alterations has in common an excessive, altered accumulation of extracellular matrix, mainly collagen, which involves a disorganization of the normal tissue architecture and, consequently, a functional loss.

In particular, hepatic fibrosis is the main complication of chronic liver damage and its progression leads to cirrhosis in the long term. The most frequent causes of hepatic fibrosis are alcohol intake, infections due to the hepatitis C virus and non-alcoholic steatohepatitis (NASH). It further contributes to a large extent to the development of hepatic insufficiency and portal hypertension.

In consequence, the evaluation of the presence and severity of hepatic fibrosis is an important parameter in clinical practice and constitutes a valuable indicator of the risk of cirrhosis progression.

Furthermore, the availability of appropriate methods to evaluate the presence and severity of fibrosis is a determining tool in the research of potentially antifibrosing molecules.

Liver biopsy and the subsequent histological examination is still today the reference technique for evaluating hepatic fibrosis. Nevertheless, it is an expensive technique with limited application, which is invasive and painful and may also cause complications for the individual's health. The precision and reproducibility of this method is seriously questioned due to the problems inherent in the tissue specimen extraction and the intra- and inter-observer variations.

Various approaches have been proposed to measure the progression such as chemical and haematological routine analysis, physiological examinations and serum fibrosis markers.

The measurement of extracellular matrix proteins or of its synthesis and degradation products in serum has been suggested, for example (US-5,316,914, EP-0283779).

US 6,631,330 proposes a method using binary logistic regression models based on a series of clinical serological parameters (markers). This scheme has been used to develop the Fibrotest (Biopredictive, Paris, France), a fibrosis index that combines the evaluation of five indirect serum fibrosis markers. This test, applied in combination with FibroScan (Echosens, Paris, France) (Sandrin L. et al. 2003. "Transient elastography: a new non-invasive method for assessment of hepatic fibrosis"; Ultrasound Med. Biol. 29: 1705-1713), seems to function suitably for the evaluation of the progression of fibrosis in hepatitis C virus infections.

WO2004/063753 proposes an evaluation method based on the analysis of serum N-glycan protein profiles.

Nevertheless, new simple methods, which are easy to perform and non-invasive and which permit appropriately evaluating the degree of hepatic fibrosis are still necessary, particularly in the less severe stages of fibrosis.
There is evidence that many pathological processes are associated with quantitative and functional changes in the molecular constituents of body fluids. As urine is an easily available body fluid, the development of a method to evaluate hepatic fibrosis based on the determination and quantification of fibrosis indicator analytes, e.g. proteins, detectable in the urine, would be very desirable and advantageous.
The object of the present invention is, therefore, the development of a method to evaluate the presence and severity of hepatic fibrosis, and also of other vital organs, by the determination and quantification of different indicator analytes detectable in a biological sample, preferably urine. To do this, the differential protein expression patterns (proteome) in the urine of healthy individuals and individuals with hepatic fibrosis have been analysed, which has permitted the selection of various proteins as candidates for biomarkers.

### BRIEF DESCRIPTION OF THE DRAWINGS

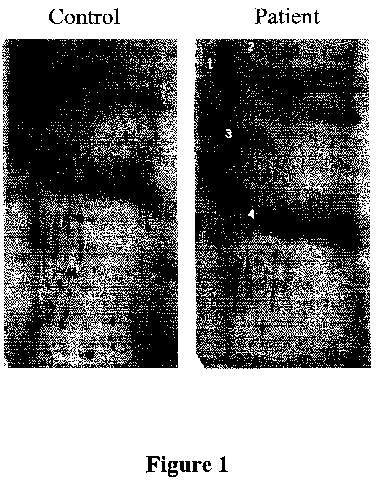

Figure 1. Identification of the increase and appearance of the proteins uromodulin, MAC2BP, AGP1 and cathepsin A in urine samples in patients with hepatic fibrosis. Representative gels from patients and control individuals are shown. The differential spots are indicated as 1: uromodulin, 2: MAC2BP, 3: AGP1 and 4: cathepsin A.

### DETAILED DESCRIPTION OF THE INVENTION

In a first aspect, the present invention relates to the use of a combination of at least two markers selected from uromodulin, MAC2BP, AGP1 and cathepsin A, for the in vitro detection of fibrotic alterations. This use is characterized in that it comprises obtaining a biological sample and measuring the concentration of said markers in the sample.

Furthermore, the invention relates to a process to evaluate in vitro the presence (or absence) and severity (stage or degree of development) of a fibrotic alteration. Said process is characterized in that it comprises:
a) obtaining a biological sample;
b) determining the levels of at least two markers in said sample, selected from:
   - uromodulin
   - MAC2BP
   - AGP1, and
   - cathepsin A.
   The term "marker" relates to a molecule or compound whose quantity is evaluated. In the method of the invention, the four markers whose levels are determined are proteins. The presence-absence or increase-decrease of said markers in the biological sample is indicative of the presence or severity of a fibrotic alteration.
   The term "uromodulin" relates to the protein coded by the gene identified in humans by the symbol UMOD (LOCUSLINK: 7369 Homo sapiens; UNIGENE: Hs. 164470), in any of its isoforms and in any animal species. Said protein is also known as Uromucoid or Tamm-Horsfall glycoprotein (THP). In a particular embodiment the protein is human protein (Swiss-Prot Accession number P07911, Last update: Release 47 10-May-2005).

The term "MAC2BP" relates to the protein coded by the gene identified in humans by the symbol LGALS3BP (LOCUSLINK: 3949 Homo sapiens; UNIGENE: Hs. 514535), in any of its isoforms and in any species. Said protein is also known as "Galectin-3 binding protein", "Mac-2 binding protein" (MAC2BP) or "Tumor-associated antigen 90K". In a particular embodiment the protein is human protein (Swiss-Prot Accession number Q08380, Last update: Release 47 10-May-2005). This is a glycoprotein that promotes cellular adhesion and may stimulate the defences against viruses and tumoral cells.

The term "AGP1" relates to a protein coded by the gene identified in humans by the symbol ORM1 (LOCUSLINK: 5004 Homo sapiens; UNIGENE: Hs. 494984), in any of its isoforms and in any species. Said protein is also known as "Alpha-1-acid glycoprotein" (AGP1), or orosomucoid 1 (OMD1). In a particular embodiment the protein is human protein (Swiss-Prot Accession number P02763, Last update: Release 47 10-May-2005). Its intervention in the modulation of immune system activity during the acute reaction phase has been described. It undergoes alterations in its glycosilation pattern in patients with cirrhosis and a promoter function of hepatic fibrosis has also been suggested. A process to diagnose liver disease (hepatitis, cirrhosis, hepatocarcinoma) using a specific antibody for this protein has been proposed (WO2004058823).

The expression "cathepsin A" relates to a protein coded by the gene identified in humans by the symbol PPGB (LOCUSLINK: 5476 Homo sapiens: UNIGENE: Hs. 517076), in any of its isoforms and in any species. Said protein is also known as "Lysosomal protective protein precursor", carboxypeptidase C, "Protective protein for beta-galactosidase" or by the code E.C. 3.4.16.5. In a particular embodiment the protein is human protein (Swiss-Prot Accession number P10619, Last update: Release 47 10-May-2005).

The biological sample where the concentration of the markers is measured may be a tissue homogenate, a tissue lysate or a biological fluid, e.g. blood, plasma, serum, urine, saliva, cerebrospinal liquid, tears, amniotic liquid and milk, etc. In a particular embodiment said biological sample is blood, plasma, serum or urine. In a preferred embodiment, said biological sample is a urine sample.

The preparation of the biological sample for its evaluation according to the present invention is performed by the conventional processes known by analysis laboratory technicians, basically depending on the type of biological sample and the configuration of the method of the invention chosen for the evaluation. In the simplest embodiments, e.g. a sample evaluation of urine using immunochromatographic strips ("immunostrip" or a "dipstrip"), the sample is applied directly with no need for preparation. When additional determinations are required, aiming the measurement of different markers or the confirmation of previous results, the evaluation can be performed in aliquots of the same sample or of different samples taken from the same subject.

In the present invention, the biological sample may come from any animal, particularly any mammalian animal, such as, for example, a laboratory animal (rodent, a rabbit, a primate, a dog), a pet or domestic animal (a dog, a cat, a horse, a bovine or porcine animal) or a wild animal. In a particular embodiment of the invention, said biological sample comes from a man or a woman.

The determination of the levels of a compound according to the present invention means that the quantity of the referred compound in the sample is evaluated (generally expressed as concentration). Said evaluation may simply be a determination of presence-absence, a semiquantitative evaluation, or more preferably a quantitative evaluation.

A particular embodiment of the invention further comprises comparing the levels of the markers determined with respect to a reference standard or value. In a specific embodiment of the invention, levels above 1.5 times the standard levels are related to the presence of fibrotic alterations. In a preferred embodiment, levels of at least twice the reference value are linked to the presence of a fibrotic alteration.

Given that the molecular mechanisms involved in fibrosis are, at least partly, common, irrespective of the tissue wherein it develops, the organ affected by the fibrotic alteration to evaluate may be any organ. For example, the fibrotic alteration to evaluate may be a pulmonary, medullar, hepatic, pancreatic, renal, cardiac or multi-organ fibrosis.

In a preferred embodiment, the combined use of the markers of the invention is to evaluate the presence and severity of hepatic fibrosis.

In a particular embodiment, the invention comprises the combined use of three markers selected from uromodulin, MAC2BP, AGP1 and cathepsin A. Preferably the levels of the four specified markers are determined.

Optionally, the present invention may include the use of other additional markers. Said additional markers may be proteins or any other type of compound (glycoproteins, peptides, small molecules, polysaccharides, nucleic acids, antibodies, etc.).

The determination of the marker levels object of the present invention may be performed by any known analytical methods, the choice whereof will basically depend on the requirements and needs that predominate over the evaluation that one wants to obtain: speed-simplicity, sensitivity and specificity, predictive value, etc.

According to different configurations of the invention, the determination may be performed among others, by colorimetric, spectrophotometric, fluorescence, chemiluminescence processes, using radioisotopes, spectrophotometric, NMR, chromatographic, electrophoretic and chemical processes, immunoassays (based on an antigen-antibody reaction) or by a combination of said methods.

In a particular embodiment, the determination is performed by mass spectrometry, e.g. tandem mass spectrometry associated with liquid chromatography (LC-MSMS) or MALDI-TOF-MS (matrix-assisted laser desorption/ionization mass spectrometry time of flight MS).

In a preferred embodiment of the invention, the determination is performed using an immunoassay. Applicable immunoassays in the method of the invention include: homogeneous assays, heterogeneous assays, enzyme immunoassays (EIA, ELISA), competitive assays, immunometric assays (sandwich), turbidimetric assays, nephelometric assays and their combinations. The applicable principles and protocols of these immunoassays are well known and thoroughly described in manuals such as "The immunoassay handbook", edited by David Wild, 2nd edition 2001, Nature Publishing group, which is included as a reference.

In a particular embodiment, said immunoassay is an ELISA or a strip immunochromatography assay ("dipstrip", "immunochromatographic strip" or "immunostrip"). In another particular embodiment, said immunoassay is performed on an antibody chip.

In the patent document US5,753,517 we can find detailed information, which should not be taken as restrictive of the present invention, on possible applicable configurations and embodiments of a quantitative immunochromatographic strip assay, both for sandwich types tests and inhibition assays. The immunochromatographic assay may also adopt many other configurations, such as those disclosed in the patent document US6,316,205, some of which permit the analysis of several different markers in the same assay and on the same sample.

For the final evaluation of the presence (or absence) or severity of the fibrotic alteration, preferably hepatic fibrosis, in the biological sample, the levels of the markers determined in the test sample (biological sample of the subject whose fibrosis we want to evaluate) is compared with pre-established reference values. When a qualitative (positive-negative) or semi-qualitative evaluation suffices, it is only necessary to establish a cut-off value for each compound measured. When a quantitative evaluation with optimized sensitivity and specificity are required, the reference value or indexes may, for example, be established with a logistic regression analysis of the discriminative values of each one of the markers measured, and subsequent construction of a logistic function that links the measurements obtained for each compound. The quality of the logistic function is analysed using ROC curves. The procedures to construct logistic functions are well known and have also been used in the evaluation of fibrotic alterations, among them hepatic fibrosis, using serological markers. These procedures are disclosed, for example, in the document, US6,631,330, which is incorporated as reference.

In a preferred embodiment of the invention markers are used for evaluation of the presence and severity of hepatic fibrosis on urine samples, and it is performed by determining the levels of at least two of the markers selected from uromodulin, MAC2BP, AGP1 and cathepsin A. In a particular embodiment, the measurements obtained for said markers are linked by a logistic function.

The object of the invention is of use, for example, in a method to select and screen drugs with potential antifibrosing activity. This process comprises a) administering the subject (preferably an animal) the drug to study; b) at different points of the study (before, during and/or after administration) take biological samples from the animal and determine the marker levels in accordance with the present invention; and c) comparing the determinations performed on the samples obtained in the different treatment phases and compared to the control animals, for example not treated.

In another application, the invention permits controlling the progression of the fibrotic state of an organ, preferably the liver, of an animal or a patient, so that the animal or the patient is subjected to different sampling and evaluation of the defined markers. In a more particular application, the invention permits evaluating the response to the antifibrosing treatment with time.

Another aspect of the invention is characterized in that the concentration of said markers is measured using specific probes for at least two of the following markers: uromodulin, MAC2BP, AGP1 and cathepsin A.

The term "probe" relates to a molecule which specifically reacts with one of the four indicated markers (uromodulin, MAC2BP, AGP1 and cathepsin A), and whose reaction may be detected, either directly or indirectly, by a marker-tracer of the reaction intensity, which may be a coloured, fluorescent, luminescent product or marker, or any other detectable marker. In a particular embodiment, the probe is a specific ligand of uromodulin, MAC2BP, AGP1 or cathepsin A, e.g. a lectin or an antibody (antibodies). In a preferred embodiment, said probe is a specific antibody, either polyclonal or monoclonal.

In another additional aspect, the invention relates to a kit to determine the levels of at least two of the markers selected from uromodulin, MAC2BP, AGP1 and cathepsin A, characterized in that it comprises specific probes for any combination of 2, 3 or 4 of said markers. In a specific embodiment, the kit contains two specific probes for two of said markers (e.g. one probe for cathepsin A and another for uromodulin; or one for MAC2BP, another for AGP1 and another for uromodulin; etc). In another embodiment, the kit contains any combination of 3 probes for 3 of said markers. In another embodiment, the kit contains at least 4 probes, one for each one of the 4 markers uromodulin, MAC2BP, AGP1 and cathepsin A. In a preferred embodiment, at least one of the probes is a specific antibody for the marker that one wants to detect and quantify. In another particular embodiment, all the kit probes are specific antibodies.

The probe or probes of the kit of the invention may be contained in the same composition or in separate compositions, adhered to a solid substrate (e.g. on a microplate for ELISA or on a strip for immunochromatography) or on several composition substrates within the same kit. In a specific embodiment, the kit is an immunoassay kit. In a preferred embodiment, the kit of the invention is a kit for ELISA. In another embodiment, the kit of the invention is a kit for immunochromatography.

In a preferred embodiment, the kit of the invention is a kit for immunochromatography which comprises at least one specific antibody (the probe) to one of the four markers of the method of the invention and at least one immunochromatographic strip or membrane. In a particular embodiment, the specific antibodies to said marker, or part thereof, may be bound to labelled particles, coating them. In a particular embodiment, said specific antibodies are embedded in a chromatographic strip or membrane. Additionally, other material or reagents, such as antibodies for internal control, either bound or not bound to labelled particles, can be adhered to the chromatographic strip.

In a particular embodiment the kit of the invention is a kit for immunochromatography that contains: two or more probes, antibodies, preferably specific for at least two or more of the markers of the method of the invention; two or more immunochromatographic strips specific for a particular marker in accordance with the specific antibody that adheres thereto; as well as other reagents and materials necessary to detect the reaction between the analyte and the specific antibody to complete an immunochromatographic assay.

The kit of the invention may also contain other optional materials and reagents e.g. secondary antibodies, buffers, diluents, colouring or tracers (fluorescent, luminescent, etc), standards, calibration controls, test cartridges, vials, bottles, tubes, needles, chromatographic strips, microplates and instructions for use.

In a particular embodiment the kit of the invention comprises a packaging with a label with the indication "for evaluation of the presence and severity of fibrosis", and more preferably "for evaluation of the presence and severity of hepatic fibrosis." In a particular embodiment it includes the indication "for determination in urine". Said indication may be replaced by other indications considered equivalent insofar as said equivalent indications implicitly mean the application or use of the kit in a method that includes, either as an intermediate step or a final result, the evaluation of the presence and/or severity of fibrosis, preferably hepatic and/or by determination in urine.

### EMBODIMENT OF THE INVENTION

The following example aims to illustrate, by nonlimiting means, the embodiment of the invention object of the present patent application.

### EXAMPLE

In this study, urine samples obtained from 11 patients with hepatic fibrosis of different degree and source were compared with urine samples obtained from 6 control individuals. The clinical determination of hepatic fibrosis was performed using the anatomopathological study of hepatic biopsy samples collected on the same day that the urine was obtained. The fibrosis index or score corresponding to the fourth aspect evaluated on the KNODELL (Knodell RG, Ishak KG, Black WC, Chen TS, Craig R, Kaplowitz N et al. Formulation and application of a numerical scoring system for assessing histological activity in asymptomatic chronic active hepatitis. Hepatology 1981 Sep-Oct; 1: 431- 435) was used for this

To collect the urine samples, the conventional clinical protocols of the source hospital were used. The urine samples of the control individuals were collected in the same way, from healthy people from the hospital.

The analysis of the urine samples of patients with fibrosis and from healthy individuals was performed using two-dimensional electrophoresis and mass spectrophotometry. To do this, approximately 50 ml of sample from patients and control individuals were analysed. The samples were concentrated using Amicon Ultra (Millipore) concentrators with cut size of 5000 Da and the urine was re-suspended in a lysis buffer containing 7M Urea, 2M thiourea, (4% vol/vol) of 3-[cholamidopropyl) dimethyl ammonium] -1-propanesulfonate, 1% (col/col) DRR and 0.5% Biolytes 3/10. The quantity of protein was determined using the Bradford analysis kit (Bio-rad), with the albumin diluted in the lysis buffer as standard. Two-dimensional electrophoresis (2DE) was performed using 100 µg of total protein. The first dimension was performed on a Protean IEF Cell from BioRad, using 17 cm IPG strips from BioRad and it was rehydrated actively, i.e. applying a voltage of 50V at a temperature of 20°C, for 12 h. The gels ran at 60,000 V h., using a voltage ramp designed by the manufacturer. The strips were equilibrated in 50mM TRIS, pH7, 6M of urea, 30% of glycerol, 2% SDS and traces of bromophenol blue. A reduction process was performed using 2% DTT and another alkylation process using 2% iodoacetamide. The strips were directly loaded on 12.5% polyacrylamide gels (18cm x 20cm x 1mm) and they were sealed with 1% agarose. The second dimension in SDS-PAGE was performed for 15 h. The gels were stained using Spyro-Ruby fluorescence stain from BioRad. The images were digitized using Molecular Imager FX from BioRad and they were analysed using the PGQUEST 7.1 program from BioRad. In this way, an average resolution of 300 proteins was obtained, and a comparison was made using PDQUEST, wherein increases or decreases of at least twice, were accepted as differences. With this criterion, 4 protein bands were detected in urine samples from fibrotic patients, whose increase or appearance was consistent in all the assays.

The samples selected were analysed using the tryptic digestion of the different proteins, followed by liquid nanochromatography coupled to a Q TOF Micro mass spectrometer using an electrospray ionization source (ESI/MS/MS) following the protocol described below. In-gel digestion was performed with 6 ng/µl of trypsin re-suspended in 50 mM of ammonium bicarbonate at 37°C overnight. The tryptic peptides produces were extracted with 1% formic acid and 2% acetonitrile. Finally, the separation of the tryptic peptides was performed on a reverse phase Atlantis, C18 , 3 µm, 75 µm x 10 cm Nano EaseTM capillary column from Waters, equilibrated with 5% acetonitrile and 0.2% formic acid. After the injection of 6 µl of sample, the column was washed for 5 min with the same buffer and the peptides were eluted using a linear gradient of 5-50% acetonitrile in 30 min at a constant flow of 0.2 µl/min. The column was coupled online to a Q-TOF Micro from Waters, using a nano-spray type ionisation source, PicoTip from Waters. The capillary temperature was 80°C and the spray voltage was 1.8-2-2 kV. The MS/MS spectrums were collected automatically depending on the datum. The three most intense ions were sequentially fragmented by collision-induced disassociation (CID) using a 2.5 isolation window and a relative collision energy of 35%. The data processing was performed using the analysis programmes MassLynx 4.0 and ProteinLynx Global Server 2 from Waters.

The analysis of the urine samples using two-dimensional electrophoresis permitted the separation of around 700 different proteins, as can be seen in the gel image shown in Figure 1. Each of the gels of the patients' samples was compared with those of the control individuals and four bands were selected for their subsequent analysis. Four of them only appeared in the patients' samples and one of them is clearly increased in those individuals. The bands from the gels produced from the 11 patients and 6 control individuals were excised and digested with trypsin, using the protocol indicated in the previous section. The tryptic digests were then analysed using MALDI-TOF mass spectrophotometry, peptide fingerprinting and LC-ESI-QUAD-TOF mass spectrometry, to be able to study said bands. Said proteins were identified as uromodulin, MAC2BP, AGP1 and cathepsin A, marked as 1, 2, 3 and 4 respectively in figure 1. The presence or absence analysis of these proteins was analysed both in the two-dimensional gels of the control individuals and in those obtained from the patients and a distribution was obtained that is shown in Table 1. As can be observed in this table, at least 2 of the markers appear in all the fibrotic patients studies, while they are not observed in the control individuals.

**Table 1. Comparison of the increase (for uromodulin) or presence (for AGP1, cathepsin A and MAC2BP) of the different proteins. Analysis by two-dimensional electrophoresis, image analysis and MALDI-TOF or ESI/MS/MS mass spectrophotometry.**

| | **Uromodulin** | **AGP1** | **Cathepsin A** | **Mac2bp** |
|---|---|---|---|---|
| **Fibrotic patients** | | | | |
| **1** | Yes | No | Yes | Yes |
| **2** | Yes | No | Yes | Yes |
| **3** | Yes | No | Yes | Yes |
| **4** | Yes | Yes | Yes | Yes |
| **5** | Yes | Yes | Yes | Yes |
| **6** | Yes | No | Yes | Yes |
| **7** | Yes | Yes | Yes | Yes |
| **8** | Yes | Yes | Yes | Yes |
| **9** | Yes | Yes | Yes | Yes |
| **10** | Yes | Yes | No | Yes |
| **11** | Yes | Yes | Yes | Yes |

| **Controls** | | | | |
|---|---|---|---|---|
| **C1** | No | No | No | No |
| **C2** | No | No | No | No |
| **C3** | No | No | No | No |
| **C4** | No | No | No | No |
| **C5** | No | No | No | No |
| **C6** | No | No | No | No |

## Claims

1. Use of a combination of at least two markers selected from uromodulin, MAC2BP, AGP1 and cathepsin A, for the detection in vitro of fibrotic alterations

2. Use according to claim 1, **characterized in that** it comprises:
a) obtaining a biological sample; and
b) measuring the concentration of said markers in the sample.

3. Use according to claim 2, **characterized in that** said biological sample is selected from blood, plasma, serum and urine.

4. Use according to any one of claims 2 or 3, **characterized in that** said biological sample comes from a mammal animal.

5. Use according to any one of claims 2 to 4, **characterized in that** said biological sample comes from a man or a woman.

6. Use according to any one of claims 1 to 5, **characterized in that** it further comprises comparing the marker levels with a reference value.

7. Use according to claim 6, **characterized in that** levels of at least twice the reference value are linked to the presence of a fibrotic alteration.

8. Use according to any one of claims 1 to 7, **characterized in that** said fibrotic alteration is selected from pulmonary, medullar, hepatic, pancreatic, renal, cardiac or multi-organ fibrosis.

9. Use according to any one of claims 1 to 8, **characterized in that** said fibrotic alteration is a hepatic fibrosis.

10. Use according to any one of claims 1 to 9, **characterized in that** said marker concentration is determined by a method selected from a colorimetric, spectrophotometric, NMR, chromatographic, electrophoretic method, an immunoassay or a combination of said methods.

11. Use according to any one of claims 1 to 10, **characterized in that** it comprises the combination of 3 of the markers specified in said claim 1.

12. Use according to any one of claims 1 to 11, **characterized in that** it comprises the combination of the 4 markers uromodulin, MAC2BP, AGP1 and cathepsin A.

13. Use according to any of claims 2 to 12, **characterized in that** the concentration of said markers is measured using specific probes for at least two of the markers.

14. Use according to claim 12, **characterized in that** at least one of the probes is a specific antibody.

15. A kit for determining the levels of at least two markers selected from uromodulin, MAC2BP, AGP1 and cathepsin A, **characterized in that** it comprises specific probes for any combination of 2, 3 or 4 of said markers.

16. A kit according to claim 15, **characterized in that** at least one of the probes is a specific antibody.

17. A kit according to any one of claims 15 or 16, **characterized in that** it is a kit for immunoassay.

18. A kit according to any one of claims 15 to 17, **characterized in that** it is a kit for immunochromatography.

19. A kit according to any one of claims 15 to 17, **characterized in that** it is a kit for ELISA.

20. A kit according to any one of claims 15 to 19, **characterized in that** it further comprises other components selected from secondary antibodies, tracers, buffers, diluents, standards, calibration controls, test cartridges, vials, chromatographic strips, microplates and instructions for use.

21. A kit according to any one of claims 15 to 20, **characterized in that** it comprises packaging with a label with the indication for the evaluation of the presence and severity of fibrosis or an equivalent indication.

22. A kit according to claim 21 with the indication for the evaluation of the presence and severity of hepatic fibrosis.

23. A kit according to claim 21 where the label also comprises the indication for the determination in urine.
